# EUROPEAN PATENT APPLICATION

(11) **EP 4 458 157 A1**
(43) Date of publication of application: **06.11.2024**
(21) Application number: 21970111.7
(22) Date of filing: 30.12.2021
(51) Int. Cl.: A23B 7/16, A23B 7/154, B01D 11/02, C07D 311/30, C07D 311/40

(54) **METHOD FOR EXTRACTING FLAVONOLS DERIVED FROM TROPICAL FRUIT WASTE TO PRESERVE FRESH AND CUT FOODS, AND COMPOSITION COMPRISING SAME**

(71) Applicant: Bio Natural Solutions S.A.C., Lima 18 (PE)
(72) Inventor: MALNATI RAMOS, Miguel Enrique Jesús, Lima 32 (PE); ORTEGA NUÑEZ, Moises Alexander, Lima 33 (PE); ARANDA CASAVERDE, Michelle Antoinette, Lima 03 (PE); RIOS MENDOZA, Jesaya Alison, Lima 31 (PE)
(74) Representative: Herrero & Asociados, S.L.
(86) International application number: PCT/PE2021/050025
(87) International publication number: WO 2023/128771

(57) **Abstract**

The present invention relates to a method for the extraction of a composition and the preparation of a colloidal dispersion comprising flavonols derived from tropical fruit residues for the preservation of fresh and sliced meals, and the composition and dispersion thus obtained comprising mainly the following steps: a) removal of macro-pollutants from tropical fruit residues b) extraction and efficient recovery of bioactive substances with eco-friendly treatments, c) purification with membrane technology and eco-friendly activated carbon, d) organic synthesis and concentration of the flavonol composition.

## Description

### TECHNICAL FIELD

The present invention includes optimal methods for the extraction of flavonols from residues (peel, seeds and bagasse) of tropical fruits, preferably mango and/or avocado, and the subsequent transformation thereof into a preservation technology for fresh meals and ready-to-eat (RTE) sliced meals. This, with the purpose of generating a natural and fungistatic effect that provides functional properties against food deterioration, protecting its freshness and extending the shelf life thereof.

The present invention is included within the technical field of food industries, bioindustry, biotechnology, bioengineering and agroindustrial derivatives.

### STATE OF THE ART

Fresh or cut fruit and vegetable products are highly perishable products due to their intense metabolic activity, so their quality is highly vulnerable to various abiotic and biotic factors. For these reasons, adequate post-harvest handling is required to guarantee their preservation to ensure export and distribution, avoiding losses, waste and deterioration over time. Nowadays, there is great interest in developing innovative and profitable ecological biotechnologies that help preserve the shelf life of fruits with limited shelf life, or of fourth range processed meals such as ready-to-eat (RTE) sliced meals, a feasible alternative being the use of biodegradable coatings and the enrichment thereof with extracts of an ecological nature.

In this sense, flavonoids, natural pigments present in various vegetables have a great antioxidant activity generating chelating and sequestering properties of free radicals, as well as inhibition of oxidases that when applied to various fruits generate a physical-chemical effect that reduces oxidation and improves the preservation of food in an unexpected way. There are several flavonoid compounds in the residues of tropical fruits such as mango such as flavones, flavonols and related molecules. There is no research that relates the application of flavonoids, specifically flavonols from tropical fruit residues such as mango or avocado for their inclusion in preparations with improved effect in agricultural products in order to enhance their agro-industrial value; however, the use of flavonols with beneficial functions for humans has been reported in different industries such as pharmaceuticals and medicine; consequently, the main flavonols reported are: quercetin and kaempferol.

Quercetin, as a flavonoid - flavonol, has antioxidant and anticarcinogenic properties, as well as effects on the regulation of gene expression. The patent ES2464192A1 contemplates phenolic extracts rich in mangiferin and quercetin in a percentage of 1.93% and 0.83% respectively, obtained by highpressure green solvents for cosmetic and/or food use. This patent contemplates 4 steps a) a raw material preparation step, b) high pressure extraction with green solvents such as supercritical carbon dioxide and water/alcohol mixtures, c) a supercritical column concentration step and d) a solvent evaporation under vacuum or with nitrogen. Patent KR101300667B1 refers to a method for separating quercetin from an onion comprising a mixing step in alcoholic lysate for the preparation of quercetin tablets.

Likewise, there is the background CN107349240A, which describes a method to extract quercetin and other polyphenols from mango peel, performing a first cleaning and drying process in oven at 55-65 °C for 10-12 h, then performs an enzymatic extraction assisted by ultrasound, between 42-54 °C for 3-5h, adds 2-5 % by weight of aluminum chloride hydrolysate, performs an ultrafiltration of the extract and concentration under vacuum no higher than 75 °C. However, this background lacks a polarity gradient soxhlet extraction with isopropanol, petroleum ether, ethanol, and/or whey, and does not perform odor reduction with activated carbon.

On the other hand, research has been reported giving quercetin antimicrobial capacity. Studies have shown that quercetin not only has a good inhibitory effect on bacteria, but also has an important inhibitory activity on fungi. Several experiments have found that quercetin has a good inhibitory effect on the growth of pathogenic bacteria such as Pseudomonas aeruginosa, Salmonella enteritidis, Staphylococcus aureus, Escherichia coli, Proteus and Aspergillus flavus (Yang et al., 2020). In turn, Hossion et al. (2011) found that novel, artificially designed and synthesized quercetin acyl glucosides effectively inhibited the growth of E. coli, S. aureus and P. aeruginosa. In addition, bayberry extract has significant antibacterial activities against Salmonella, Listeria and Shigella with minimum inhibitory concentration (MIC) values ranging from 2.07 to 8.28 mg / ml.

In this sense, the extraction of this flavonol is of great importance due to the chemical and biological properties thereof.

Kaempferol, a flavonol antioxidant found in fruits and vegetables, has dietary effects that reduce the risk of chronic diseases, especially cancer. Therefore, patent CN105924419A provides a method to extract kaempferol from camellia oleifera leaves following steps of: leaching, D101 macroporous adsorption resin separation, separation by silica gel chromatography and DAC chromatographic separation that are combined with chromatographic analysis. As to patent CN104557833A, the invention describes a method for extracting kaempferol using peach blossoms. The method comprises the steps of: performing pretreatment, defatting, extraction, concentration, hydrolysis and purification operations on the peach blossoms to be treated to obtain a kaempferol product.

On the other hand, regarding studies on antioxidants included in edible coatings or technologies applied in agribusiness, Pedro (2019) reports a study based on a mango starch coating with phenolic additives extracted by the Soxhlet method that imparted active properties (antioxidant and UV absorbing) to the films, although they also decreased the transparency to visible light of the films. Another study by Leon (2018) reports an investigation on the generation of edible films formulated with mango peel which showed good barrier properties, with water vapor permeability. However, the addition of antioxidant extract based on polyphenols does not show a significant effect (p > 0.05) on the optical properties, nor performs a comprehensive study on the shelf life granted.

On the other hand, some studies have been reported on the use of avocado residues and extracts to formulate edible coatings, for example; Ramesh et al (2021) investigated the preparation of edible films using avocado seed starch, where they performed the analysis and modeling of the response surface for water vapor permeability, the results showed that the sorbitol and Tween-20 used, significantly affect the vapor property of the edible film based on avocado seed starch, therefore the individual use of the coating is recommended to enhance its effectiveness.

Therefore, no references have been found that relate the use of flavonols from tropical fruit residues, such as mango and/or avocado to enrich coatings or films for the preservation of fresh produce or RTE sliced meals, Therefore, it is necessary to have a process for the efficient extraction of flavonols with high antioxidant and chelating power from tropical fruit residues, and easy to process without organic chemical additives in the final product, which contribute to preserve and/or improve the nutritional and organoleptic features in fresh food and RTE sliced meals, intended for humans and animals, with food grade.

In addition, the valorization of agro-industrial waste will help us to reduce the environmental impact and take advantage of certain molecules such as flavonols to use them in the elaboration of coatings with bioactive power.

### DESCRIPTION OF THE INVENTION

As a solution to the technical problem mentioned above, the present invention was developed which proposes a novel method for the extraction of a flavonol composition, the method for the preparation of its colloidal dispersion and to the products obtained in said methods, from tropical fruit residues, preferably fruits such as mango and avocado, and residues such as peels, pips and bagasse of the fruits, wherein the compound comprises at least 75% of the compounds quercetin and kaempferol concentrated in the composition.

The invention also relates to a process for the preparation of a solution and/or coating with protective effect for fresh and cut fruit and vegetable products, such as fruits, vegetables, tubers and roots, and their subsequent extension of shelf life.

The present invention contains flavonols having phenyl-benzo-γ-pyrone (or phenyl-γ-chromone) structure, products of the secondary metabolism of tropical fruit residues that we use as raw material. They are found in the form of heterosides. These heterosides are soluble in aqueous solvent, while the genins or aglycones thereof, the non-sugar part of the heteroside, are soluble to a lesser extent. The flavonols of this invention are constituted by a 3-ring structure with a diphenyl propane backbone of formula I.

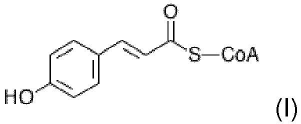

This single ring is synthesized by the condensation of 3 moles of malonyl-coenzyme A from glucose metabolism and generates a chalcone synthase of formula II.

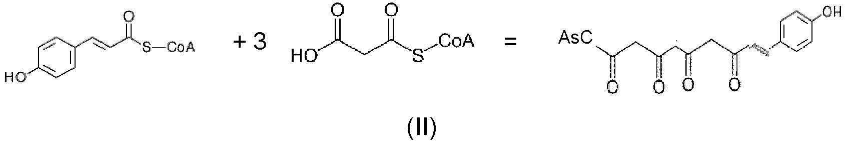

As a basic structure, flavonoids have a carbon backbone C6-C3-C6 - formula III, where the C6 components are aromatic rings linked by three carbon atoms that may or may not form a third pyran or pyrone ring (A-C rings). The different classes of flavonoids have different concentrations of saturation and in the C-ring substituents, while the individual compounds, within each of these groups, are distinguished by the different substitution of the A and B rings. Thus, we have identified 2 flavonols and 1 flavonoid in our patent.

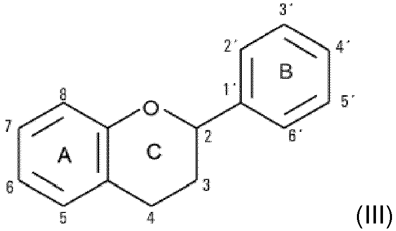

From this distribution, our invention isolated quercetin present as (2-(3-(3,4-dihydroxyphenyl)-3,5,7-trihydroxy-4H-chromen-4-one) of formula IV.

The biosynthesis of quercetin initially passed through the phenylpropanoid pathway, where phenylalanine was converted to 4-coumaryl-CoA by the catalytic action of phenylalanine ammonia lyase, cinnamate-4-hydroxylase and 4-coumaryl CoA ligase. Then, chalcone synthase catalyzed the combination of 4-coumaroyl-CoA with three molecules of malonyl-CoA (3), resulting in the formation of tetrahydroxy chalcone. Tetrahydroxy chalcone was converted to naringenin using chalcone isomerase and naringenin was converted to eriodictyol by the action of the enzyme flavanone-3β-hydroxylase. The eriodictyol formed was converted to dihydroquercetin by the action of flavanone-3β-hydroxylase, which was finally converted to quercetin of formula IV by flavonol synthase.

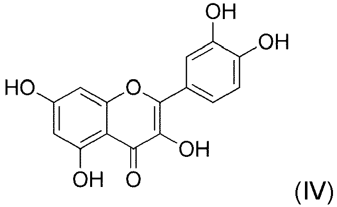

The production of kaempferol begins when phenylalanine is converted to p-coumaryl-CoA by phenylalanine ammonia lyase (PAL), cinnamic acid 4-hydroxylase (C4H) and 4-coumaric acid ligase (4CL). Then, naringenin is generated by condensation reaction between a molecular p-coumaryl-CoA and three molecules of malonyl-CoA by synthesis of chalcone (CHS) and chalcone isomerase (CHI). Finally, naringenin is converted to kaempferol via dihydrokaempferol by flavanone 3β-hydroxylase (F3H) and flavonol synthase (FLS).

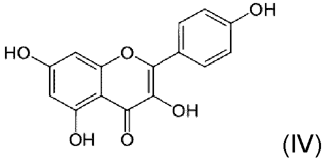

Finally, with the present invention, from the by-products, a liquid active compound, formula (V), high in quercetin and kaempferol, was extracted in combination.

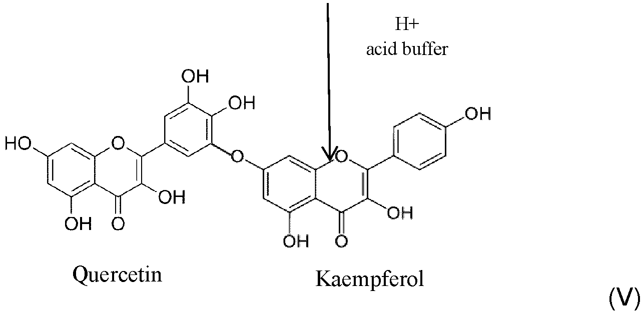

The object of the invention is the method for the extraction of a concentrated flavonol composition and the preparation of the colloidal dispersion thereof, from tropical fruits preferably mango and avocado, and residues such as peels, seeds and bagasse, comprising the following steps a) removal of macro-pollutants from tropical fruit residues b) extraction and efficient recovery of bioactive substances with ecological treatments, c) purification with membrane technology and ecological activated carbon, d) organic synthesis and concentration of the flavonol composition.

The removal of macro-pollutants from tropical fruit residues such as peels, seeds and bagasse, comprises the following sub-processes in detail:
- Selection, washing and disinfection of tropical fruit residues such as peels, seeds and selected bagasse.
- Partial dehydration of the raw material with heat assistance, preferably to be carried out in a conventional oven at 40-60 °C for about 3-6 hours with hot air reflux.
- Continuous extraction using soxhlet equipment at 80 °C for 3-6 cycles, using solutions of organic solvents with water in a graded ratio of 1:1 to 1:30, with Isopropanol, petroleum ether, ethanol, or whey as a medium to remove macromolecules in a ratio between 40%-60%.

Efficient extraction and recovery of bioactive substances with environmentally friendly treatments is performed by pre-treatment with ultrasound with a vibration power between 50 - 80 kHz, for 30 - 60 minutes at a temperature between 40 - 60 °C, followed by convection heat assisted extraction at 40-60 °C for a ratio of 2-6 hours, using solutions in proportion between 20-70% ethanol in ratio solvent raw material between 1:10 - 1:30, based on the nature and state of maturation of the raw material.

The purification with membrane technology and eco-friendly activated carbon of tropical fruit waste extract comprises the use of tangential pumping to a sequential filter battery of particle size 10 - 200 nm and an activated carbon column to retain typical odors, at a pumping pressure at 0.5-1 bar. Previously, environmentally friendly activated carbon is obtained from conditioning filtered solid residues of tropical fruits, preferably mango, with a 25-30% phosphoric acid solution carbonized at 700-800 K for a time of 2-4 hours.

The organic synthesis and concentration of the flavonol composition is carried out by a preliminary mixture of the tropical fruit residue extract and a reaction with an acid medium such as phosphate buffer (45 nM), followed by vacuum distillation in a rotary evaporator under vacuum between 0.5 - 15 inHg at 40-65 °C for 30 - 90 minutes until a concentration between 20-40% is achieved.

The flavonol composition of tropical fruit residues can be applied directly on fruit and vegetable products, through a solution with purified water in a 1:10 - 1:30 ratio, depending on the nature of the product.

The object of the invention also comprises the preparation of a colloidal dispersion and/or coating with organic barrier function for fresh and cut fruit and vegetable products, such as fruits, vegetables, tubers and roots, and the subsequent extension of shelf life thereof, thanks to the chelating and antioxidant effect of efficiently synthesized and concentrated flavonols, comprising the following postulates:
- Suspend and/or dissolve between 5-20% of the flavonol composition of tropical fruit residues in a colloidal medium such as sodium alginate, xanthan gum, gum arabic, guar gum, sapote gum and/or any related gum in a proportion between 3 - 6%, together with additives that complement its barrier effect such as food grade plasticizers between 3-6%, saturated fatty acid or waxes of natural origin such as carnauba wax, beeswax or candelilla wax in a proportion between 5-10%.
- Formation of microemulsions of postulate b, from high revolutions above 10 500 RPM for an efficient application on the surface of the fruits.
- Dehydration and/or pulverization of the colloidal suspension of postulate c for its manipulation in solid form.

An advantage of the present invention is that obtaining the flavonol composition involves an alternative with an efficiency above 75% of the flavonol compounds quercetin and kaempferol.

Another advantage of the invention is that it supposes alternatives for using flavonols as relevant technologies for maintaining the quality of many fresh and cut fruit and vegetable products in time, not described in the state of the art, and their enrichment with active agents from the residues of tropical fruits such as mango and avocado, for their integral use in the productive process of fresh and processed meals.

An objective of the invention is to obtain flavonol antioxidants of high phenolic concentration, from tropical fruit residues, preferably mango and avocado, with chelating effect directly linked to their effect on the shelf life of fresh produce and perishable sliced meals.

The invention represents an improvement in the state of the art, since no use of flavonols as bioactive compounds for food preservation has been reported, even less extracted from agro-industrial wastes, which represents a contribution to the problem of food losses and wastes at a global level, and to the use of ecological, circular and sustainable solutions that are so necessary in the world to fight against global warming.

### BRIEF DESCRIPTION OF THE FIGURES

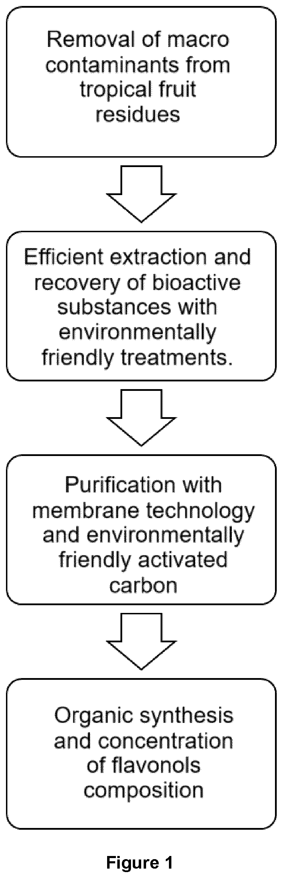

**Figure 1****.** Flow diagram of the process of the invention showing four main steps thereof.
**Figure 2****.** Rot index (%) in tangerines at 49 days of storage.
**Figure 3****.** Soluble Solids Dynamics (°Brix) in persimmons at 21 days of storage.
**Figure 4****.** Minimum Inhibitory Concentration of the formulated invention.

### PREFERRED EXAMPLES (OR PREFERRED EMBODIMENTS OF THE INVENTION)

Procedure for the extraction of a compound comprising flavonols derived from tropical fruit residues for the preservation of fresh and sliced meals, and the application thereof.

A preparation of the extraction of the compound comprising flavonols derived from tropical fruit residues, for the preservation of fresh meals of the invention, by way of example, involves the selection, washing and disinfection of the peels, seeds and bagasse of the fruit of mango Mangifera indica L. with 100 ppm of sodium hypochlorite diluted in filtered water; then proceed to dehydrate with a tray dehydrator at a temperature of about 40 °C for 6 hours. Once the dehydration is completed, the moisture content of the raw material is measured and purified with organic solvents such as 40 % isopropanol at a ratio of 1:20 by means of assisted and continuous extraction with soxhlet (apparatus for extracting substances of low solubility in the extraction solvent, employing a solid-liquid separation technique) for up to 5 cycles; then the raw materials follow an ultrasound pre-treatment with a vibration power between 80 kHz, for 40 minutes at a temperature between 50 °C for 1 hour to extract the bioactive compounds, purifying them through two sequential filters with nanopores of 10 nm and minimum surface of 0.01 m², at the same temperature of the previous process, through which the pumped solution passes at pressures between 0.5 bar, and an ecological activated carbon column made from the bagasse of the previous filtered solids carbonized and conditioned with 25% phosphoric acid, which provides odor adsorption and a circular process of the integral use of the waste. The solution is mixed with a 45 nM Buffer Phosphate solution to pH <3.5 for 1 hour and concentrated by vacuum distillation method in an evaporator under pressure between 0.5 - 15 inHg at 65°C for 60 minutes to a concentration of at least 20%.

In a separate process, 10% of the composition comprising flavonols derived from mango residues is suspended in a solution of sodium alginate 5% glycerol USP (2%), saturated fatty acid (5%), and distilled water with constant motion at 500 RPM for 1 hour and homogenized at 12000 RPM forming a microemulsion which is part of the preferred example applied on fresh post-harvest agricultural products such as tangerine and persimmon, from the spray method applying 1 liter of product per ton of agricultural product, being non-exclusive. The results at 49 days under ideal storage conditions for fresh tangerine and 21 days for fresh persimmon and cut pineapple, as well as the antioxidant and fungistatic power of the flavonol composition, were presented below:

### Evaluation of the invention on the postharvest life of cut horticultural products (RTE).

Experiments were conducted to evaluate the effect of the invention on the shelf life of sliced meals, such as ready-to-eat pineapples, previously peeled and chopped compared to an untreated control. The experiments were then stored under refrigeration (Table 1 and 2).

### Evaluation of the invention on the postharvest life of fresh horticultural products

Experiments were conducted to evaluate the effect of the invention on the postharvest life of tangerines (Citrus reticulata) and persimmons (Diospyrus kaki) in comparison to a control with polyethylene wax in the case of tangerines, and without coating as a negative control in the case of persimmons (Table 2). The coating treatment concerning the invention was applied by spraying on the surface of the fruits and left to dry at room temperature. Subsequently, the experimental units were stored under specific conditions to simulate export conditions (Table 1).

**Table 1. Fruit storage conditions**

| **Sample** | **Cold days** | **Cold temperature** | **Reference** |
|---|---|---|---|
| Tangerine *(Citrus reticulata*) | 49 | 9 °C | Lo'ay, A. A., & Dawood, H. D. (2019). Tolerance of 'Baladi'tangerine fruits to cold storage by postharvest pectin/PVA blend with ascorbic acid treatment. Scientia Horticulturae, 256, 108637. |
| Kaki *(Diospyros kaki*) | 21 | 12°C | Saleem, M. S., Ejaz, S., Anjum, M. A., Nawaz, A., Naz, S., Hussain, S., ... & Canan, I. (2020). Postharvest application of gum arabic edible coating delays ripening and maintains quality of persimmon fruits during storage. Journal of Food Processing and Preservation, 44(8), e14583. |
| Cut pineapple *(Ananas comosus L.)* | 8 | 10°C | Azarakhsh, N., Osman, A., Ghazali, H. M., Tan, C. P., & Adzahan, N. M. (2014). Lemongrass essential oil incorporated into alginate-based edible coating for shelf-life extension and quality retention of fresh-cut pineapple. Postharvest Biology and Technology, 88, 1-7. |

**Table 2. Summary of results**

| **Sample** | **Control** | | **Patent of Invention** | |
|---|---|---|---|---|
| | **% Weight loss** | **% of rust** | **% Weight loss** | **% of rust** |
| Tangerine (*Citrus reticulata*) | 12.78% | 1.5% | 8.51% | 0.5% |
| Kaki (*Diospyros kaki*) | 7.45% | 2.0% | 2.9% | 0.0% |
| Cut pineapple (Ananas comosus L.) | 14.5% | 12% | 6.2% | 1.0% |

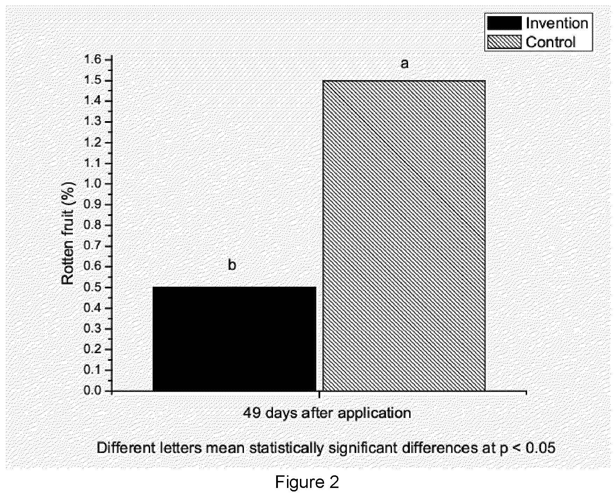

The application of the coating referred to the invention generates, statistically, a lower weight loss in tangerines stored in cold storage. Bioactive coatings of other chemical natures have the same or lower performance in this parameter compared to other polyethylene waxes (Arnon et al., 2014). Rot control is effective compared to polyethylene coatings (See Figure 2).

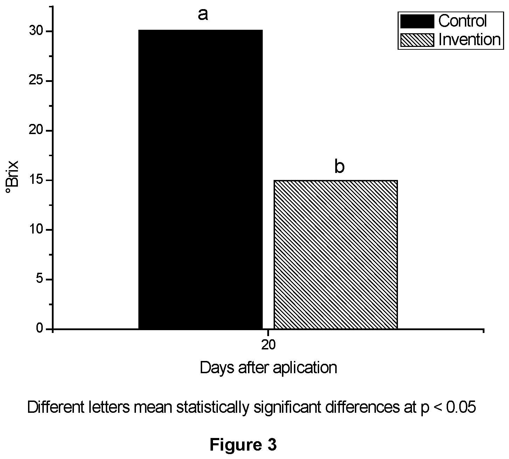

The application of the coating referring to the invention generates, statistically, a lower weight loss in cold stored kakis. The use of edible coatings, as reported by Saleem et al. (2020), delays weight loss in persimmon fruits. The results of this experiment show a lower weight loss with the coating referring to the invention compared to the coating reported by the aforementioned authors. On the other hand, there is the same trend in the reduction of soluble solids increase, with similar figures in both studies (See Figure 3).

On the other hand, Azarakhsh (2014) reported a weight loss of more than 14% in untreated pineapples and greater than 9% for cut pineapples applied with an alginate-based coating, which is greater than that reported by the invention at 8 days of evaluation.

### Antioxidant power of the invention

The methods of quantification of bioactive compounds were performed by spectrophotometric techniques, among them, the method of quantification of total flavonoids (CFT), and total phenolic compounds (CPT).

The present invention develops efficient methods of extraction of bioactives, resulting in a rate of 780.75 ± 1.3 to 1126.22 ± 1.9 mg GAE /g* for CPT and 815.94±0.46 to 1429.21±0.78 mg QE/g** of flavonoids. The minimum and maximum values are a function of the extraction parameters used for the recovery of the bioactive compounds, mentioned above.

Recent research on avocado and/or mango peel shows that traditional extraction technologies recover phenolic compounds up to 10%. Kemadjou et al., (2021), evaluated the ethanol extract of 12 avocado peel varieties and reported values from 733 to 1037 mg GAE/g (CPT); 311 to 724 mq QE/g for total flavonoids. Likewise, Aminah et al., (2016), reported rates of 4.012 mgQE/g in ethanol extracts; Trujillo-Mayo et al. (2018), 257.2+- 7.515 mg GAE/g and 57.1 +- 0.9 mq QE/g in extracts macerated for 12 hours. On the other hand, emerging technologies based on microwave extraction (MAE), ultrasound (U) and the mixture of both, have achieved higher recovery of phenolic compounds, as a reference of this, Mariane et al, (2018), reached values of 1360.34 +- 188.65 mg QE/g in aqueous extracts; Trujillo-Mayo et al. (2018), 281.4 +- 0.2 mg GAE/g and 62 +- 0.4 mq QE/g in U-MAE extracts; Tremocoldi et al., (2017), 120.3 - 7.8 mg GAE/g in U extracts with freeze-drying treatment of the sample.

### *milligrams of gallic acid per gram of dry sample

### **milligrams of standard quercetin per gram of dry sample

### Fungistatic power

To evaluate the fungistatic power of the present invention, in vitro tests were performed on phytopathogenic fungi Cladosporium herbarum and Aspergillus niger.

| **Strain evaluated** | **Storage temperature** | **Days of storage** |
|---|---|---|
| Cladosporium herbarum | 25°C | 7 |
| Aspergillus niger | 25°C | 7 |

### In vitro tests

The strains of C. herbarum and A. niger were seeded in Petri dishes with Sabouraud Agar, and 60 uL of the formulated invention (mixture of flavonoids, as a positive control Belomil and distilled water, as a negative control) were placed.

The assays are performed in triplicate at least twice, thus obtaining 3 treatments and 18 plates in total for each of the fungi.

Finally, the plates were incubated at 25°C for seven days, taking as a positive result the appearance of an inhibition halo around the wells. The clear zones of growth inhibition (halos) were observed and the diameters in mm were measured with the Vernier caliper, recorded and considered as an indication of antifungal activity.

The results were reported in Tables 3 and 4:

**Table 3. Statistical analysis of the mean inhibition halos of A. niger.**

| Treatment | N | Media | SD | HSD |
|---|---|---|---|---|
| Invention | 6 | 30.33 mm | 0.427 | a |
| C+ | 6 | 15.73 mm | 0.763 | b |
| C- | 6 | 0.0 mm | 0 | c |

Inv. invention of the formulation; C+. Belomil; C-. water; N. number of replicates; SD. Standard deviation.

*Tukey; different letters in columns represent significant differences at 5% error.

**Table 4. Statistical analysis of the mean inhibition halos of C. herbarum.**

| Treatment | N | Media | SD | HSD |
|---|---|---|---|---|
| Inv | 6 | 31.33 mm | 0.578 | a |
| C+ | 6 | 15.4 mm | 0.681 | b |
| C- | 6 | 0.0 mm | 0 | c |

Inv. invention of the formulation; C+. Belomil; C-. water; N. number of replicates; SD. Standard deviation.

*Tukey; different letters in columns represent significant differences at 5% error.

With these results, the formulation of the invention was subjected to the micro dilution plate test to determine its Minimum Inhibitory Concentration (MIC).

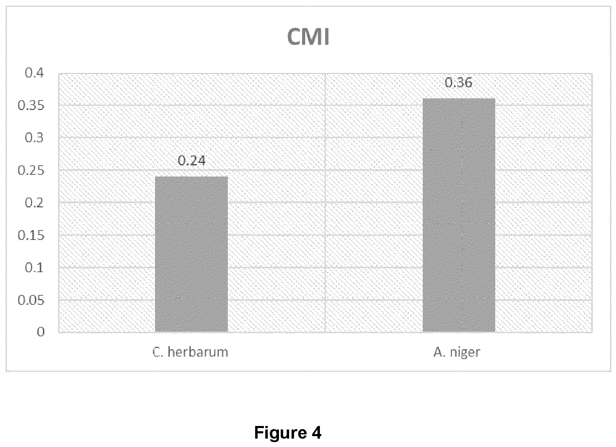

Figure 4 shows the MIC results of the formulated invention against Aspergillus niger and Cladosporium herbarum phytopathogens. The concentrations of the invention ranged from 10 mg/ml to 0.02 mg/ml. Finally, the MIC for A. niger was 0.36 mg/ml and 0.24 mg/ml for C. herbarum.

The formulated invention showed effective antifungal activity with a MIC of 0.24 mg/ml for Cladosporium herbarum, a result that presented highly significant advantages compared to several studies that confronted extracts to the mentioned strain; for example, Gonzales et. al (2016) evaluated three concentrations of the plant extract (25, 50, and 75 %) of the species Moringa oleifera Lam. (Moringa), Morinda citrifolia L. (noni), Azaridachta indica A. Juss (neem), Melia azedarach L. (paradise), where only the paradise extract showed an effective result to inhibit Cladosporium spp at the concentration of 25 %. As for Aspergillus niger, the present study reported an MIC of 0.36 mg/ml result which differs advantageously with various studies, such as that of Diaz et al (2019) who reported an MIC of 3.12 mg/ml for garlic crude extract.

## Claims

1. An extraction method of a composition comprising flavonols derived from tropical fruit residues for the preservation of fresh and sliced meals, **characterized by** comprising the following steps:
a) removal of macro-pollutants from tropical fruit residues through: disinfection of said residues, dehydration of said residues, continuous extraction with heat for 3-6 cycles, using organic solvent solutions with water in a graded ratio between 1:1 to 1:30, with Isopropanol, petroleum ether, ethanol, or whey as a means to remove macromolecules in a proportion by weight between 40%-60%;
b) extraction and efficient recovery of bioactive substances with environmentally friendly treatments of the product of step a), carried out through: an ultrasound pre-treatment, followed by a convection heat assisted extraction, using solutions in a weight ratio between 20-70% ethanol in a solvent/raw material ratio between 1:10 - 1:30;
c) purification with membrane technology and ecological activated carbon of the extract from the previous step;
d) organic synthesis and concentration of the flavonol composition from the extract of the previous step.

2. The extraction method of a composition comprising flavonols derived from tropical fruit residues for preserving fresh and sliced meals according to claim 1, wherein the step of removing macro-pollutants from the residues comprises the following sub-steps in detail:
- before disinfection, the tropical fruit residues are sorted and washed;
- partial dehydration of the residues is carried out with heat assistance at 40-60 °C for about 3-6 hours with hot air reflux;
- for continuous extraction, equipment is used to extract substances of low solubility in the extraction solvent (soxhlet) at 80 °C for 3-6 cycles, using solutions of organic solvents with water in a graded ratio of 1:1 to 1:30, with isopropanol, petroleum ether, ethanol, or whey as a means to eliminate macromolecules in a proportion by weight between 40%-60%.

3. The extraction method of a composition comprising flavonols derived from tropical fruit residues for preserving fresh and sliced meals according to claim 1, wherein the step of extracting and efficiently recovering bioactive substances with environmentally friendly treatments comprises the following sub-steps in detail:
- ultrasound pre-treatment is performed with a vibration power between 50 - 80 kHz, for 30 - 60 minutes at a temperature between 40 - 60 °C,
- convection heat assisted extraction is carried out at 40-60 °C for 2-6 hours, using solutions in a weight ratio between 20-70% ethanol in a solvent to raw material ratio between 1:10 - 1:30, depending on the nature and step of maturation of the raw material.

4. The extraction method of a composition comprising flavonols derived from tropical fruit residues for the preservation of fresh and sliced meals according to claim 1, wherein the purification step with membrane technology and environmentally friendly activated carbon comprises the use of tangential pumping to a sequential filter battery of particle size 10 - 200 nm and an activated carbon column to retain typical odors, at a pumping pressure at 0.5-1 bar.

5. The extraction method of a composition comprising flavonols derived from tropical fruit residues for the preservation of fresh and sliced meals, according to claim 1, wherein the environmentally friendly activated carbon is obtained from conditioning the filtered solid tropical fruit residues with a phosphoric acid solution 25-30% by weight carbonized at 700-800 K for a time of 2-4 hours.

6. The extraction method of a composition comprising flavonols derived from tropical fruit residues for the preservation of fresh and sliced meals, according to claim 1, wherein the organic synthesis and concentration step of the flavonol composition is performed by: a preliminary mixing of the tropical fruit residue extract and a reaction with an acid medium such as Buffer Phosphate (45 nM), then proceeding to vacuum distillation in a rotary evaporator subjected to vacuum between 0.5 - 15 inHg at 40-65°C for 30 - 90 minutes until a concentration between 20-40% by weight is achieved.

7. The extraction method of a composition comprising flavonols derived from tropical fruit residues for the preservation of fresh and sliced meals according to claim 1, **characterized in that** the flavonols are derived from husk, seed and/or bagasse residues, from fruits such as mango and avocado, and wherein the formulation obtained is:

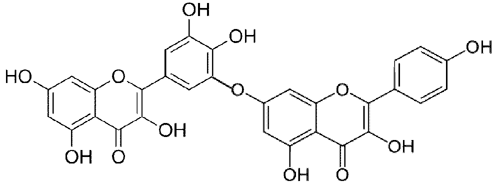

8. A composition comprising flavonols derived from tropical fruit residues for fresh and sliced meals preservation, obtained via the method of claim 1, **characterized in that** the concentrated extract comprises at least 75% by weight of concentrated quercetin and kaempferol compounds in the composition.

9. A method for the preparation of a colloidal dispersion comprising flavonols derived from tropical fruit residues for the preservation of fresh and sliced meals, **characterized by** comprising the following steps:
a) removal of macro-pollutants from tropical fruit residues through: disinfection of said residues, dehydration of said residues, continuous extraction with heat for 3-6 cycles, using organic solvent solutions with water in a graded ratio between 1:1 to 1:30, with Isopropanol, petroleum ether, ethanol, or whey as a means to remove macromolecules in a proportion by weight between 40%-60%;
b) extraction and efficient recovery of bioactive substances with environmentally friendly treatments of the product of step a), carried out through: an ultrasound pre-treatment, followed by a convection heat assisted extraction, using solutions in a weight ratio between 20-70% ethanol in a solvent/raw material ratio between 1:10 - 1:30;
c) purification with membrane technology and ecological activated carbon of the extract from the previous step;
d) organic synthesis and concentration of the flavonol composition from the extract of the previous step;
e) suspending and/or dissolving between 5-20% by weight of the flavonol composition of tropical fruit residues from the previous step in a colloidal medium such as sodium alginate, xanthan gum, gum arabic, guar gum, sapote gum and/or any related gum in a proportion between 3 - 6% by weight, together with additives that complement their barrier effect such as food grade plasticizers between 3-6% by weight, saturated fatty acid or waxes of natural origin such as carnauba wax, beeswax or candelilla wax in a proportion between 5-10% by weight;
f) formation of microemulsions from the previous step, from high revolutions above 10 500 rpm for an efficient application on the surface of the fruits.
g) dehydration and/or pulverization of the colloidal suspension of the previous step for its manipulation in solid form.

10. A colloidal dispersion comprising flavonols derived from tropical fruit residues for the preservation of fresh and sliced meals, prepared by the method of claim 9, **characterized by** comprising:
- between 5-20% by weight of flavonol composition of tropical fruit residues;
- between 3-6% by weight of a colloidal medium such as sodium alginate, xanthan gum, gum arabic, guar gum, sapote gum and/or any related gums;
- between 3-6% by weight of additives that complement its barrier effect such as food grade plasticizers; and
- between 5-10% by weight of saturated fatty acid or waxes of natural origin such as carnauba wax, beeswax or candelilla wax.
